# EUROPÄISCHE PATENTSCHRIFT

(11) **EP 2 620 412 B1**
(45) Veröffentlichungstag und Bekanntmachung des Hinweises auf die Patenterteilung: **25.11.2015**
(21) Anmeldenummer: 13151468.9
(22) Anmeldetag: 16.01.2013
(51) Int. Cl.: C01B 33/02, C01B 33/021, C01B 33/035, C30B 13/00, C23C 16/24, G01J 3/28, G01N 21/64

(54) **VERFAHREN ZUR BESTIMMUNG EINER OBERFLÄCHEN-VERUNREINIGUNG VON POLYKRISTALLINEM SILICIUM**
PROCESS FOR DETERMINING SURFACE CONTAMINATION OF POLYCRYSTALLINE SILICON
Procédé de détermination d'une impureté de surface de silicium polycristallin

(30) Priorität: 24.01.2012 DE 102012200994
(43) Veröffentlichungstag der Anmeldung: 31.07.2013
(73) Patentinhaber: Wacker Chemie AG, 81737 München (DE)
(72) Erfinder: Wochner, Hanns Dr., 84489 Burghausen (DE); Baumann, Robert Dr., 84489 Burghausen (DE)
(74) Vertreter: Potten, Holger

(56) Entgegenhaltungen:
- EP-A2- 0 345 618
- DE-A1- 4 137 521
- DE-A1-102006 035 081
- DE-A1-102008 040 231

## Beschreibung

Gegenstand der Erfindung ist ein Verfahren zur Bestimmung einer oberflächlichen Verunreinigung von polykristallinem Silicium.

Im industriellen Maßstab wird Rohsilicium durch die Reduktion von Siliciumdioxid mit Kohlenstoff im Lichtbogenofen bei Temperaturen von etwa 2000 °C gewonnen.

Dabei erhält man sog. metallurgisches Silicium (Si_{mg}, "metallurgical grade") mit einer Reinheit von etwa 98-99 %.

Für Anwendungen in der Photovoltaik und in der Mikroelektronik muss das metallurgische Silicium gereinigt werden. Dazu wird es beispielsweise mit gasförmigem Chlorwasserstoff bei 300-350 °C in einem Wirbelschichtreaktor zu einem Silicium enthaltenden Gas, beispielsweise Trichlorsilan, umgesetzt. Darauf folgen Destillationsschritte, um das Silicium enthaltende Gas zu reinigen.

Dieses hochreine Silicium enthaltende Gas dient dann als Ausgangsstoff für die Herstellung von hochreinem polykristallinem Silicium.

Das polykristalline Silicium, oft auch kurz Polysilicium genannt, wird üblicherweise mittels des Siemens-Prozesses hergestellt. Dabei werden in einem glockenförmigen Reaktor ("Siemens-Reaktor") dünne Filamentstäbe aus Silicium durch direkten Stromdurchgang erhitzt und ein Reaktionsgas enthaltend eine Silicium enthaltende Komponente und Wasserstoff wird eingeleitet.

Die Silicium enthaltende Komponente des Reaktionsgases ist in der Regel Monosilan oder ein Halogensilan der allgemeinen Zusammensetzung SiHₙX₄₋ₙ (n=0, 1, 2, 3; X = Cl, Br, I). Bevorzugt handelt es sich um ein Chlorsilan, besonders bevorzugt um Trichlorsilan. Überwiegend wird SiH₄ oder SiHCl₃ (Trichlorsilan, TCS) im Gemisch mit Wasserstoff eingesetzt.

Beim Siemens-Prozess stecken die Filamentstäbe üblicherweise senkrecht in am Reaktorboden befindlichen Elektroden, über die der Anschluss an die Stromversorgung erfolgt. Je zwei Filamanentstäbe sind über eine waagrechte Brücke (ebenfalls aus Silicium) gekoppelt und bilden einen Trägerkörper für die Siliciumabscheidung. Durch die Brückenkopplung wird die typische U-Form der auch Dünnstäbe genannten Trägerkörper erzeugt.

An den erhitzten Stäben und der Brücke scheidet sich hochreines Polysilicium ab, wodurch der Stabdurchmesser mit der Zeit anwächst (CVD = Chemical Vapour Deposition / Gasphasenabscheidung).

Nach Beendigung der Abscheidung werden diese Polysiliciumstäbe üblicherweise mittels mechanischer Bearbeitung zu Bruchstücken unterschiedlicher Größenklassen weiterverarbeitet, gegebenenfalls einer nasschemischen Reinigung unterzogen und schließlich verpackt.

Das Polysilicium kann aber auch in Form von Stäben oder Stabstücken weiterverarbeitet werden. Dies gilt insbesondere für Verwendung des Polysiliciums in FZ-Verfahren.

Daneben ist es auch bekannt, kleine Siliciumpartikel in einem Wirbelschichtreaktor direkt einem solchen Reaktionsgas auszusetzen. Das dabei erzeugte polykristalline Silicium liegt in Form von Granulaten vor (Granulat-Poly).

Polykristallines Silicium (kurz: Polysilicium) dient als Ausgangsmaterial bei der Herstellung von einkristallinem Silicium mittels Tiegelziehen (Czochralski- oder CZ-Verfahren)- oder mittels Zonenschmelzen (Floatzone oder FZ-Verfahren). Dieses einkristalline Silicium wird in Scheiben (Wafer) zertrennt und nach einer Vielzahl von mechanischen, chemischen und chemo-mechanischen Bearbeitungen in der Halbleiterindustrie zur Fertigung von elektronischen Bauelementen (Chips) verwendet.

Insbesondere wird aber polykristallines Silicium in verstärktem Maße zur Herstellung von ein- oder multikristallinem Silicium mittels Zieh- oder Gieß-Verfahren benötigt, wobei dieses ein- oder multikristalline Silicium zur Fertigung von Solarzellen für die Photovoltaik dient.

Da die Qualitätsanforderungen an Polysilicium immer höher werden, sind Qualitätskontrollen während der gesamten Prozesskette unabdingbar. Untersucht wird das Material beispielsweise hinsichtlich Kontaminationen mit Metallen oder Dotierstoffen. Zu unterscheiden ist die Kontamination im Bulk und die Kontamination an der Oberfläche der Polysiliciumbruchstücke oder -stabstücke.

Es ist üblich, das erzeugte Polysilicium zum Zwecke der Qualitätskontrolle in einkristallines Material zu überführen. In diesem Fall wird das einkristalline Material untersucht. Auch hier spielen Metallkontaminationen, die bei den Kundenprozessen in der Halbleiterindustrie besonders kritisch einzuschätzen sind, eine besondere Rolle. Untersucht wird das Silicium aber auch hinsichtlich Kohlenstoff sowie Dotierstoffen wie Aluminium, Bor, Phosphor und Arsen.

Dotierstoffe (B, P, As, Al) werden nach SEMI MF 1398 an einem aus dem polykristallinen Material erzeugten FZ-Einkristall (SEMI MF 1723) mittels Photolumineszenz analysiert.

Alternativ kommt Niedrigtemperatur-FTIR (Fourier-Transform IR-Spektroskopie) zum Einsatz (SEMI MF 1630).

Grundlagen des FZ-Verfahrens sind beispielsweise in der DE-3007377 A beschrieben.

Beim FZ-Verfahren wird ein polykristalliner Vorratsstab mit Hilfe einer Hochfrequenzspule nach und nach aufgeschmolzen und das schmelzflüssige Material durch Animpfen mit einem einkristallinen Impflingskristall und anschließendem Rekristallisieren in einen Einkristall überführt. Bei der Rekristallisation wird der Durchmesser des entstehenden Einkristalls zunächst kegelförmig vergrößert (Konusbildung) bis ein gewünschter Enddurchmesser erreicht ist (Stabbildung). In der Phase der Konusbildung wird der Einkristall auch mechanisch gestützt, um den dünnen Impflingskristall zu entlasten.

Von dem aus einem polykristallinen Siliciumstab mittels FZ erzeugten einkristallnen Stab wird eine Scheibe (Wafer) abgeschnitten (SEMI MF 1723). Aus dem gezogenen einkristalline Stab wird ein Scheibchen herausgeschnitten, mit HF/HN03-geätzt, gespült mit 18 MOHm Wasser und getrocknet. An dieser Scheibe werden die Photolumineszenzmessungen durchgeführt.

FTIR (SEMI MF 1188, SEMI MF 1391) ermöglicht die Bestimmung von Kohlenstoff- und Sauerstoffkonzentrationen.

Dabei wird aus einem polykristallinen Stab ein Scheibchen herausgeschnitten. Das Scheibchen wird poliert. Anschließend wird mittels FTIR-Spektroskopie der Kohlenstoffgehalt bestimmt.

Beide Verfahren (Photolumineszenz) und FTIR dienen ausschließlich der Bestimmung von Verunreinigungen im Bulk.

Verunreinigungen an der Oberfläche können nur indirekt bestimmt werden.

DE 10 2008 040231 A1 offenbart die Herstellung von polykristallinem Siliciumbruch hoher Reinheit sowie ein Reinigungsverfahren. Proben von Polysiliciumbruch können aufgearbeitet und einem analytischen Verfahren zur Bestimmung des Metallgehalts der Bruchstückoberflächen unterzogen werden.

DE 10 2006 035081 A1 offenbart ein Verfahren zur Herstellung von hochreinem klassierten Polysilicium-Bruch, bei dem ein Polysilicium aus dem Siemens-Verfahren mittels einer Vorrichtung umfassend Zerkleinerungswerkzeuge und einer Siebvorrichtung zerkleinert und klassiert wird und der so erhaltene Polysilicium-Bruch mittels eines Reinigungsbades gereinigt wird, wobei die Zerkleinerungswerkzeuge und die Siebvorrichtung durchgängig eine mit dem Polysilicium in Kontakt kommende Oberfläche aus einem Werkstoff besitzen, welches den Polysilicium-Bruch nur mit solchen Fremdpartikeln verunreinigt, welche anschließend gezielt durch das Reinigungsbad entfernt werden.

EP 0 345 618 B1 offenbart einen polykristallinen Siliciumstab mit weniger als 15 ppta Bor und weniger als 20 ppta Phosphor. Aus einem derartigen Stab kann mittels Schwebezonenverfahren einkristallines Silicium mit einem spezifischen Widerstand von mindestens 10.000 Ohmcm und einer Lebensdauer von mindestens 10.000 µs erzeugt werden.

DE 41 37 521 A1 beschreibt ein Verfahren zur Analyse der Konzentration an Verunreinigungen in Siliciumteilchen, dadurch gekennzeichnet, dass teilchenförmiges Silicium in ein Siliciumgefäss gegeben, das teilchenförmige Silicium und das Siliciumgefäss zu monokristallinem Silicium in einer Schwebezone aufarbeitet und die Konzentration an Verunreinigungen, die in dem monokristallinen Silicium vorhanden sind, bestimmt werden. Die Konzentrationen an Bor, Phosphor, Aluminium und Kohlenstoff im verwendeten Siliciumgefäß wurden bestimmt und liefern einen reproduzierbaren Hintergrundwert. Die nach dem Schwebezonenverfahren mittels FTIR ermittelten Werte an Bor, Phosphor und Kohlenstoff wurden korrigiert um den Anteil, der aus dem Siliciumgefäß stammte. In dieser Anmeldung wird auch gezeigt, dass die Fragmentierung eines polykristallinen Siliciumstabes zu einer Kontamination des Siliciums führt. Dies ist möglich, indem Siliciumfragmente in das Siliciumgefäß gegeben, dem Schwebezonenverfahren unterzogen und anschließend mittels FTIR auf Verunreinigungen untersucht werden. Da die Kontamination des Grundmaterials vor Fragmentierung bekannt ist, kann auf die zusätzliche Kontamination durch die Fragmentierung geschlossen werden.

DE 43 30 598 A1 offenbart ebenfalls ein Verfahren, das es ermöglicht, auf die Verunreinigung von Silicium infolge von Zerkleinerungsprozessen zu schließen. Ein Siliciumblock wurde in Brocken zerbrochen. Der Siliciumbrocken wurde anschließend einem Zonenschmelzverfahren unterzogen und in einen Einkristall überführt. Vom Einkristall wurde ein Wafer herausgesägt und mittels Photolumineszenz auf Bor und Phosphor untersucht. Gegenüber dem durchschnittlichen Bor- und Phosphorgehalt des verwendeten Siliciumblocks zeigt sich eine Erhöhung der Bor- und Phosphor-Konzentration, die u.a. auch auf den Zerkleinerungsprozess zurückzuführen ist.

Die beschriebenen Verfahren berücksichtigen jedoch nicht, dass auch die Umgebung, in der die nicht nur der Zerkleinerungsprozess, sondern auch andere Prozessschritte wie Lagerung, Transport, Reinigen, Verpacken stattfinden, Einfluss auf die Kontamination des Siliciums, insbesondere auf dessen oberflächliche Kontamination, haben.

Ein rein analytisches Verfahren zu Testzwecken ist diesbezüglich unzureichend.

Aus der beschriebenen Problematik ergab sich die Aufgabenstellung der Erfindung.

Die Aufgabe wird gelöst durch ein Verfahren zur Bestimmung einer Oberflächenverunreinigung von polykristallinem Silicium, umfassend die Schritte
a) Bereitstellen von zwei polykristallinen Siliciumstäben durch Abscheidung in einem Siemensreaktor;
b) Verunreinigungen mit Kohlenstoff und Dotierstoffen im ersten der beiden Stäbe werden unmittelbar nach der Abscheidung bestimmt, wobei eine Scheibe von diesem Stab abgetrennt und zur Bestimmung der Kohlenstoffkonzentration mittels FTIR verwendet wird, wobei ein nach Abtrennen der Scheibe von diesem Stab verbleibender Stab mittels FZ in einen einkristallinen Stab überführt wird, wobei an einer vom einkristallinen Stab abgetrennten Scheibe mittels Photolumineszenz die Konzentration von Dotierstoffen bestimmt wird;
c) Der zweite Stab wird durch eine oder mehrere Anlagen geführt, in denen polykristalline Siliciumstäbe zu Stabstücken oder Polysiliciumbruchstücken weiterverarbeitet, gegebenenfalls gereinigt, gelagert oder verpackt werden;
d) Daraufhin werden Verunreinigungen mit Kohlenstoff und Dotierstoffen im zweiten Stab bestimmt, wobei der Stab mittels FZ in einen einkristallinen Stab überführt wird, von diesem einkristallinen Stab eine erste Scheibe abgetrennt und einer FTIR-Messung zur Bestimmung der Kohlenstoffkonzentration zugeführt wird, wobei eine zweite Scheibe abgetrennt und einer Photolumineszenz-Messung zur Bestimmung der Konzentration an Dotierstoffen zugeführt wird;
wobei sich aus der Differenz der bestimmten Verunreinigungen mit Kohlenstoff und Dotierstoffen in erstem und zweitem Stab eine Oberflächenverunreinigung von polykristallinem Silicium mit Kohlenstoff und Dotierstoffen infolge Anlagen und Anlagenumgebung ergibt.

Zunächst werden zwei polykristalline Siliciumstäbe bereitgestellt, indem in einem Siemens-Reaktor polykristallines Silicium abgeschieden wird, wobei U-förmige polykristalline Siliciumkörper, umfassend jeweils zwei polykristalline Siliciumstäbe, entstehen.

Als Reaktionsgas werden bei der Abscheidung typischerweise eine Silicium enthaltende Komponente, vorzugweise Trichlorsilan, und Wasserstoff verwendet.

Vorzugsweise erfolgt die Abscheidung in einem kleinen Testreaktor.

In der Praxis kann ein zuvor genannter U-förmiger Körper nach der Abscheidung aus dem Reaktor ausgebaut, wobei anschließend Brücke und die jeweiligen Stabenden abgetrennt werden, so dass zwei polykristalline Siliciumstäbe ein- und derselben Charge gewonnen werden.

Die beiden in Schritt a) bereitgestellten polykristallinen Siliciumstäbe waren vorzugsweise während der Abscheidung über eine Brücke (U-Form) miteinander verbunden (Bruderstäbe).

Bei Verwendung eines kleinen Reaktors können die beiden polykristallinen Siliciumstäbe typischerweise eine Länge von etwa 20 cm und einen Durchmesser von etwa 1,6 cm aufweisen.

Einer der beiden Stäbe wird vorzugsweise unmittelbar nach der Abscheidung und dem Abtrennen von Brücke und Stabende in einen PE-Beutel verpackt. Vorzugsweise werden beide Stäbe jeweils in einen PE-Beutel verpackt.

Dieser erste Stab wird anschließend hinsichtlich Kontaminationen untersucht.

Es werden Verunreinigungen mit Dotierstoffen sowie Kohlenstoff bestimmt.

Vorzugsweise wird in einem analytischen Labor, zu dem der verpackte Stab transportiert wird, der Stab aus dem PE-Beutel entnommen, eine Scheibe vom polykristallinen Stab abgetrennt und der FTIR-Messung zugeführt.

Dabei wird die Kohlenstoffkonzentration bestimmt.

Der verbleibende Stab wird mittels FZ in einen einkristallinen Stab überführt.

An diesem wird mittels Photolumineszenz die Konzentration von Dotierstoffen bestimmt.

Die so ermittelten Werte für Dotierstoff- und Kohlenstoffkonzentrationen dienen als Referenzwerte für den zweiten Stab.

Der zweite Stab wird nach Entnahme aus dem PE-Beutel vorzugsweise durch die Anlagen zur Erzeugung von polykristallinem Siliciumbruch (Zerkleinern, Verpacken) und optional durch die Anlagen zur Reinigung von polykristallinem Siliciumbruch geführt.

Hierbei nimmt der Stab die Verunreinigungen bezüglich Dotierstoffen und Kohlenstoff beim Durchfahren der Anlagen auf.

Nach dem Durchfahren der Reinigungsanlagen oder der Straße zur Erzeugung von ungereinigtem Polybruch wird der kontaminierte Stab vorzugsweise wiederum in einen hochreinen PE-Beutel verpackt.

Auf den PE-Beutel werden vorzugsweise zwei Etiketten aufgeklebt
Etikett Nr. 1: Etikett mit der Chargennummer der Orginalcharge (Vergleich mit Erststab)
Etikett Nr. 2: Etikett mit einer neuen Chargennummer

Aus dem kontaminierten Stab wird mittels FZ ein einkristalliner Stab erzeugt.

Anschließend werden - wie oben für den ersten Stab beschrieben - Dotierstoffe mittels Photolumineszenz und Kohlenstoff mittels FTIR bestimmt.

Im Unterschied zum ersten Stab erfolgt die Bestimmung der Kohlenstoffkonzentration mittels FTIR nicht an einer polykristallinen, sondern an einer einkristallinen Scheibe.

Beim FZ-Ziehen des kontaminierten Stabes wandern die Kohlenstoffhaltigen Partikel von der Oberfläche in den Bulk und werden damit der Kohlenstoffmessung mittels FTIR zugänglich.

Die beim ersten Stab gemessenen Werte werden von den Werten des zweiten, durch die Anlagen geführten Stabs abgezogen.

Die Differenzwerte zwischen dem ersten und dem zweiten Stab ergeben dann den Wert, der der Oberfläche des polykristallinen Siliciums nach Bearbeitung, Reinigung, Verpackung zugewiesen werden kann.

Das erfindungsgemäße Verfahren bietet somit die Möglichkeit, indirekt zu bestimmen, wie Polysilicium bei den Bearbeitungsschritten wie Zerkleinern, Reinigen, Verpacken oder bei Transportvorgängen an der Oberfläche kontaminiert wird.

Das Verfahren liefert somit Oberflächenkontaminationen für alle möglichen Produkte, wie Polysiliciumstäbe, Cutrods und Polysiliciumbruch unterschiedlicher Größenklassen (geätzt oder nicht geätzt).

Das Verfahren ermöglicht es auch, einzelne Herstellungsschritte hinsichtlich Oberflächenkontaminationen zu überwachen und zu optimieren:
Beispielsweise kann der zweite Stab nur durch die Reinigungsanlage oder nur durch die Zerkleinerungsanlage geführt werden. Dann liefert das Verfahren separat den Einfluss der Zerkleinerungsanlage und dessen Umgebung oder der Reinigungsanlage und dessen Umgebung auf die Oberflächenkontamination. Gleiches gilt für das Verpacken von Polysilicium oder den Transport der Polysiliciums von der einen Anlage zu einer anderen Anlage.

Die Bestimmung der Oberflächenverunreinigung ist reproduzierbar.

Zur Überprüfung wurden zwölf Stäbe in zwölf Prozessschalen zum gleichen Zeitpunkt durch die Reinigungsanlage gefahren.

Anschließend wurden die Dotierstoffkonzentrationen mittels Photolumineszenz ermittelt.

Theoretisch müssten die zwölf Bruderstäbe - obwohl sie aus unterschiedlichen Chargen stammen - die gleichen Analysenwerte bezüglich Bor, Phosphor, Aluminium und Arsen aufweisen, da sie gleichzeitig unter den gleichen Bedingungen durch die Reinigungsanlage gefahren wurden.

**Tabelle 1** zeigt die ermittelten Werte für Bor, Phosphor, Aluminium und Arsen in ppta.

Die beim ersten Stab gemessenen Werte wurden von den Werten des zweiten, durch die Anlagen geführten Stabs abgezogen.

**Tabelle 1**

| | B | P | Al | As |
|---|---|---|---|---|
| #1 | 20,95 | 23,37 | 0,98 | 4,95 |
| #2 | 12,74 | <1 | <0,5 | <0,5 |
| #3 | 14,40 | 1,25 | <0,5 | 2,04 |
| #4 | 16,04 | 5,49 | <0,5 | 0,52 |
| #5 | 20,96 | 10,09 | <0,5 | <0,5 |
| #6 | 17,79 | 7,22 | 0,52 | <0,5 |
| #7 | 12,28 | <1 | <0,5 | <0,5 |
| #8 | 14,03 | <1 | <0,5 | <0,5 |
| #9 | 22,15 | 13,85 | <0,5 | 1,60 |
| #10 | 21,98 | 7,51 | <0,5 | 2,03 |
| #11 | 12,49 | 1,52 | <0,5 | 1,71 |
| #12 | 22,91 | 14,86 | 0,50 | <0,5 |

Es konnten folgende Reproduzierbarkeiten und Nachweisgrenzen abgeschätzt werden:
**Bor**
   Reproduzierbarkeit: +/- 5 ppta
   Nachweisgrenze: 5 ppta
**Phosphor**
   Reproduzierbarkeit: +/- 5 ppta
   Nachweisgrenze: 5 ppta
**Aluminium**
   Reproduzierbarkeit: +/- 0,25 ppta
   Nachweisgrenze: 1 ppta
**Arsen**
   Reproduzierbarkeit: +/- 0,5 ppta
   Nachweisgrenze: 5 ppta

Mit dem erfindungsgemäßen Verfahren kann auch der Gehalt an Kohlenstoffpartikeln auf der Siliciumoberfläche mit einer Reproduzierbarkeit von +/- 10 ppba bei einer Nachweisgrenze von 10 ppba bestimmt werden.

### Beispiel

Im Beispiel wird gezeigt, wie der zweite Stab durch die Reinigungsanlage geführt und anschließend hinsichtlich Dotierstoffkonzentration untersucht wird. Der erste Stab (Bruderstab des zweiten Stabs aus einem U-förmigen Körper nach Abscheidung) wurde - wie oben beschrieben -mittels Photolumineszenz hinsichtlich Dotierstoffen analysiert.

Der PE-Beutel, in den der zweite Stab (Länge 20 cm, Durchmesser 1,6 cm) verpackt wurde, wird mit einer Schere, vorzugsweise einer Keramikschere, geöffnet. Der Stab wird entnommen, wobei zur manuellen Entnahme ein hochreiner Handschuh benutzt wird. Anschließend wird der Stab in eine Prozessschale gelegt.

Ein geeigneter hochreiner Handschuh (PE-Tyvek^{®} Handschuh) ist offenbart in der US 2011-0083249, auf die hier in vollem Umfang Bezug genommen wird. Bei Tyvek^{®} der Firma DuPont handelt es sich um ein papiervliesartiges Faserfunktionstextil aus thermisch verschweißten Fasern aus Polyethylen mit hoher Dichte (HDPE).

Die mit dem Stab befüllte Prozessschale wird durch die Reinigungsanlage gefahren.

Dabei wird der Siliciumstab in einer Vorreinigung mit einer oxidierenden Reinigungslösung gewaschen, die die Verbindungen Flurwasserstoffsäure (HF), Chlorwasserstoffsäure (HCl) und Wasserstoffperoxid (H₂O₂) enthält. In einer Hauptreinigung wird der Stab mit einer Reinigungslösung gewaschen, die Salpetersäure (HNO₃) und Fluorwasserstoffsäure (HF) enthält. Anschließend wird der Stab mit einer oxidierenden Reinigungslösung gewaschen und damit hydrophiliert. Bezüglich des Reinigungsverfahrens wird in vollem Umfang auf EP 0 905 796 B1 Bezug genommen.

Nach dem Reinigen des Stabs wird dieser getrocknet und nach Abkühlen mit einem hochreinen Handschuh, vorzugsweise einem PE-Tyvek^{®} Handschuh angefasst und in einen hochreinen PE-Beutel verpackt und verschweißt.

Auf den PE-Beutel werden zwei Etiketten aufgeklebt:
Etikett Nr. 1: Etikett mit der Chargennummer der Orginalcharge (Ermöglicht den Vergleich der Messwerte mit Erststab)
Etikett Nr. 2 Etikett mit einer neuen Chargennummer

Der kontaminierte Stab wird mittels FZ zu einem einkristallinen Stab verarbeitet. Wie oben beschrieben werden Dotierstoffe mittels Photolumineszenz bestimmt. Ebenso könnte mittels FTIR auch Kohlenstoff untersucht werden.

Die für den ersten Stab gemessenen Werte für die Dotierstoffe Bor, Phosphor, Aluminium und Arsen werden von den entsprechenden Werten für den zweiten Stab abgezogen.

Die Differenzwerte zwischen dem ersten und dem zweiten Stab ergeben dann die Werte, die der Oberfläche des Polysiliciums zugewiesen werden können.

**Tabelle 2** zeigt die ermittelten Differenzwerte für Oberflächenkontaminationen an Bor, Phosphor, Aluminium und Arsen.

**Tabelle 2**

| **B** | **P** | **Al** | **As** |
|---|---|---|---|
| 44,06 | 15,46 | 0,03 | 1,29 |
| 119,32 | 405,97 | 194,63 | 22,78 |
| 19,10 | 4,28 | 0,89 | 4,66 |
| 128,55 | 250,91 | 145,57 | 17,18 |
| 7,70 | 79,68 | 0,87 | 0,52 |
| 3,58 | 21,01 | 1,53 | 2,66 |
| 3,86 | 16,17 | 6,71 | 4,39 |
| 6,57 | 0,22 | 0,25 | 1,24 |
| 9,11 | 2,68 | 1,37 | 1,08 |
| 10,10 | 1,37 | 14,60 | 0,59 |
| 20,47 | 41,02 | 7,26 | 3,18 |

## Patentansprüche

1. Verfahren zur Bestimmung einer Oberflächenverunreinigung von polykristallinem Silicium, umfassend die Schritte
a) Bereitstellen von zwei polykristallinen Siliciumstäben durch Abscheidung in einem Siemensreaktor;
b) Verunreinigungen mit Kohlenstoff und Dotierstoffen im ersten der beiden Stäbe werden unmittelbar nach der Abscheidung bestimmt, wobei eine Scheibe von diesem Stab abgetrennt und zur Bestimmung der Kohlenstoffkonzentration mittels FTIR verwendet wird, wobei ein nach Abtrennen der Scheibe von diesem Stab verbleibender Stab mittels FZ in einen einkristallinen Stab überführt wird, wobei an einer vom einkristallinen Stab abgetrennten Scheibe mittels Photolumineszenz die Konzentration von Dotierstoffen bestimmt wird;
c) Der zweite Stab wird durch eine oder mehrere Anlagen geführt, in denen polykristalline Siliciumstäbe zu Stabstücken oder Polysiliciumbruchstücken weiterverarbeitet, gegebenenfalls gereinigt, gelagert oder verpackt werden;
d) Daraufhin werden Verunreinigungen mit Kohlenstoff und Dotierstoffen im zweiten Stab bestimmt, wobei der Stab mittels FZ in einen einkristallinen Stab überführt wird, von diesem einkristallinen Stab eine erste Scheibe abgetrennt und einer FTIR-Messung zur Bestimmung der Kohlenstoffkonzentration zugeführt wird, wobei eine zweite Scheibe abgetrennt und einer Photolumineszenz-Messung zur Bestimmung der Konzentration an Dotierstoffen zugeführt wird;
wobei sich aus der Differenz der bestimmten Verunreinigungen mit Kohlenstoff und Dotierstoffen in erstem und zweitem Stab eine Oberflächenverunreinigung mit Kohlenstoff und Dotierstoffen von polykristallinem Silicium infolge Anlagen und Anlagenumgebung ergibt.

2. Verfahren nach Anspruch 1, wobei die Dotierstoffe ausgewählt werden aus der Gruppe Bestehend aus Bor, Phosphor, Aluminium und Arsen.

3. Verfahren nach Anspruch 1 oder nach Anspruch 2, wobei der erste Stab unmittelbar nach der Abscheidung in einen PE-Beutel verpackt wird.

4. Verfahren nach einem der Ansprüche 1 bis 3, wobei es sich bei der einen oder den mehreren Anlagen in Schritt c) um Anlagen zur Zerkleinerung, zur Reinigung, zur Lagerung oder zur Verpackung von Polysilicium handelt und wobei der Stab, nachdem er durch die eine oder durch die mehreren Anlagen geführt wurde, in einen PE-Beutel verpackt wird.

## Claims

1. Process for determining surface contamination of polycrystalline silicon, comprising the steps of
a) providing two polycrystalline silicon rods by deposition in a Siemens reactor;
b) determining contamination with carbon and dopants in the first of the two rods immediately after the deposition, wherein a wafer is removed from said rod and used to determine the carbon concentration by means of FTIR, wherein a rod remaining after removal of the wafer from said rod is converted by means of FZ to a monocrystalline rod, and the concentration of dopants is determined by means of photoluminescence on a wafer removed from the monocrystalline rod;
c) conducting the second rod through one or more systems in which polycrystalline silicon rods are processed further to give rod pieces or polysilicon fragments, optionally cleaned, stored or packed;
d) then determining contamination with carbon and dopants in the second rod, wherein the rod is converted by means of FZ to a monocrystalline rod, a first wafer is removed from this monocrystalline rod and sent to an FTIR analysis for determination of the carbon concentration, and a second wafer is removed and sent to a photoluminescence analysis for determination of the concentration of dopants; wherein the difference in the contamination with carbon and dopants determined in the first and second rods gives surface contamination with carbon and dopants of polycrystalline silicon resulting from systems and the system environment.

2. Process according to Claim 1, wherein the dopants are selected from the group consisting of boron, phosphorus, aluminum and arsenic.

3. Process according to Claim 1 or according to Claim 2, wherein the first rod is packed in a PE bag immediately after the deposition.

4. Process according to any of Claims 1 to 3, wherein the one or more systems in step c) are systems for comminution, for cleaning, for storage or for packaging of polysilicon, and wherein the rod, once it has been conducted through the one or more systems, is packed in a PE bag.

## Revendications

1. Procédé de détermination d'une impureté de surface de silicium polycristallin, comprenant les étapes suivantes:
a) préparation de deux barres de silicium polycristallin par précipitation dans un réacteur Siemens;
b) des impuretés contenant du carbone et des substances dopantes dans la première des deux barres sont déterminées immédiatement après la précipitation, dans lequel un disque est découpé de cette barre et utilisé pour la détermination de la concentration en carbone par FTIR, dans lequel une barre restant après le découpage d'un disque de cette barre est convertie par FZ en une barre monocristalline, dans lequel la concentration en substances dopantes est déterminée sur un disque découpé de cette barre monocristalline par photoluminescence;
c) la deuxième barre est conduite à travers une ou plusieurs installations, dans lesquelles des barres de silicium polycristallin sont transformées en morceaux de barre ou en fragments de polysilicium, éventuellement nettoyées, entreposées ou emballées;
d) on détermine ensuite des impuretés contenant du carbone et des substances dopantes dans la deuxième barre, dans lequel la barre est convertie par FZ en une barre monocristalline, un premier disque est découpé de cette barre monocristalline et est envoyé à une mesure FTIR pour la détermination de la concentration en carbone, dans lequel un deuxième disque est découpé et envoyé à une mesure par photoluminescence pour la détermination de la concentration en substances dopantes; dans lequel une impureté de surface contenant du carbone et des substances dopantes de silicium polycristallin dues à des installations et à l'environnement d'installations découle de la différence entre les impuretés contenant du carbone et des substances dopantes déterminées dans la première barre et dans la deuxième barre.

2. Procédé selon la revendication 1, dans lequel les substances dopantes sont choisies dans le groupe composé du bore, du phosphore, de l'aluminium et de l'arsenic.

3. Procédé selon la revendication 1 ou la revendication 2, **caractérisé en ce que** la première barre est emballée immédiatement après la précipitation dans un sac de PE.

4. Procédé selon l'une quelconque des revendications 1 à 3, dans lequel ladite/lesdites installation(s) à l'étape c) sont des installations de broyage, de nettoyage, d'entreposage ou d'emballage de polysilicium, et dans lequel la barre est emballée dans un sac de PE après avoir été conduite à travers ladite ou lesdites installation(s).
